# EUROPÄISCHE PATENTSCHRIFT

(11) **EP 1 831 134 B1**
(45) Veröffentlichungstag und Bekanntmachung des Hinweises auf die Patenterteilung: **01.10.2008**
(21) Anmeldenummer: 05821923.9
(22) Anmeldetag: 19.12.2005
(51) Int. Cl.: C07C 5/333, C07C 11/167

(54) **VERFAHREN ZUR HERSTELLUNG VON BUTADIEN AUS N-BUTAN**
METHOD FOR PRODUCING BUTADIENE FROM N-BUTANE
PROCEDE DE PRODUCTION DE BUTADIENE A PARTIR DE N-BUTANE

(30) Priorität: 21.12.2004 DE 102004061514
(43) Veröffentlichungstag der Anmeldung: 12.09.2007
(73) Patentinhaber: BASF SE, 67056 Ludwigshafen (DE)
(72) Erfinder: CRONE, Sven, 67117 Limburgerhof (DE); KLANNER, Catharina, 68163 Mannheim (DE); SCHINDLER, Götz-Peter, 68219 Mannheim (DE); DUDA, Mark, 67071 Ludwigshafen (DE); BORGMEIER, Frieder, 68163 Mannheim (DE)
(74) Vertreter: Isenbruck, Günter
(86) Internationale Anmeldenummer: PCT/EP2005/013659
(87) Internationale Veröffentlichungsnummer: WO 2006/066848

(56) Entgegenhaltungen:
- WO-A-20/04007408

## Beschreibung

Die Erfindung betrifft ein Verfahren zur Herstellung von Butadien aus n-Butan.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (AcrylnitrilButadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an.

Nachteilig an der Erzeugung von Butadien im Crackprozess ist, dass zwangsläufig größere Mengen an unerwünschten Koppelprodukten anfallen.

Aufgabe der Erfindung ist es, ein Verfahren zur Herstellung von Butadien aus n-Butan bereitzustellen, bei dem in möglichst geringem Umfang Koppelprodukte anfallen.

Gelöst wird die Aufgabe durch ein Verfahren zur Herstellung von Butadien aus n-Butan mit den Schritten:
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Buten, Butadien, Wasserstoff, gegebenenfalls Wasserdampf, gegebenenfalls Kohlenstoffoxide und gegebenenfalls Inertgase erhalten wird;
C) Einspeisung des Gasstroms b und eines sauerstoffhaltigen Gases in mindestens eine zweite Dehydrierzone und oxidative Dehydrierung von 1-Buten und 2-Buten, wobei ein Gasstrom c enthaltend n-Butan, Butadien, Wasserstoff, Wasserdampf, gegebenenfalls Kohlenstoffoxide und gegebenenfalls Inertgase erhalten wird;
D) Kompression in mindestens einer ersten Kompressionsstufe und Abkühlung des Gasstroms c, wobei mindestens ein Kondensatstrom d1 enthaltend Wasser und ein Gasstrom d2 enthaltend n-Butan, Butadien, Wasserstoff, Wasserdampf, gegebenenfalls Kohlenstoffoxide und gegebenenfalls Inertgase erhalten wird;
E) Auftrennung des Gasstroms d2 durch Extraktivdestillation in einen im Wesentlichen aus Butadien bestehenden Produktstrom e1 und einen n-Butan, Wasserstoff, Wasserdampf, gegebenenfalls Kohlenstoffoxide und gegebenenfalls Inertgase enthaltenden Strom e2;
F) gegebenenfalls Kompression in einer oder mehreren weiteren Kompressionsstufen und Abkühlung des Gasstroms e2, wobei mindestens ein Kondensatstrom f1 enthaltend n-Butan und Wasser und ein Gasstrom f2 enthaltend n-Butan, Wasserstoff, gegebenenfalls Kohlenstoffoxide und gegebenenfalls Inertgase erhalten werden, und gegebenenfalls Rückführung des Kondensatstroms f1 in die erste Dehydrierzone;
G) Auftrennung des Gasstroms f2 in einen Rückführstrom g1 enthaltend n-Butan und einen Abgasstrom g2 enthaltend Wasserstoff, gegebenenfalls Kohlenstoffoxide und gegebenenfalls Inertgase durch in Kontakt bringen des Gasstroms f2 mit einem hoch siedenden Absorptionsmittel und anschließender Desorption der in dem Absorptionsmittel gelösten Gasbestandteile.

Das erfindungsgemäße Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. Durch die Koppelung von nicht-oxidativer katalytischer Dehydrierung und oxidativer Dehydrierung wird eine hohe Butadien-Ausbeute erzielt. Das Verfahren ist im Vergleich zur Erzeugung von Butadien durch Cracken durch eine hohe Selektivität gekennzeichnet. Es fallen keine Koppelprodukte an. Es entfällt die aufwendige Abtrennung von Butadien aus dem Produktgasgemisch des Crackprozesses.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₆⁺-Kohlenwasserstoffen. Die Zusammensetzung von LPG kann stark schwanken. Vorteilhafter Weise enthält das eingesetzte LPG mindestens 10 Gew.-% Butane.

Alternativ kann ein veredelter C₄-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom kann einer Isomerisierung unterworfen werden. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether eingesetzt werden.

Der n-Butan enthaltende Einsatzgasstrom a enthält im Allgemeinen mindestens 60 Gew.-% n-Butan, vorzugsweise mindestens 90 Gew.-% n-Butan. Daneben kann er als Nebenbestandteile noch C₁-C₄-Kohlenwasserstoffe enthalten.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien (1,3-Butadien) gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden. Vorzugsweise wird sie als autotherme nicht-oxidative katalytische Dehydrierung unter Einspeisung von Sauerstoff als Co-Feed durchgeführt. Bei der autothermen Fahrweise wird die benötigte Wärme direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden. Sauerstoff kann als Rein-Sauerstoff oder als sauerstoffhaltiges Gas eingespeist werden, beispielsweise als Luft. Bevorzugtes Sauerstoffhaltiges Gas ist Luft oder mit Sauerstoff angereicherte Luft mit einem Sauerstoffgehalt bis 70 Vol.-%, vorzugsweise bis 50 Vol.-%. Um den Inertgasanteil zu begrenzen, kann Sauerstoff auch als sauerstoffreiches Gas, beispielsweise mit einem Sauerstoffgehalt von mindestens 75 Vol.-% oder mindestens 90 Vol.-% eingespeist werden.

Ein Merkmal der nicht-oxidativen Fahrweise gegenüber einer oxidativen Fahrweise ist, dass bei der oxidativen Dehydrierung kein freier Wasserstoff gebildet wird.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Kataiysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet.

Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne sauerstoffhaltigem Gas als Co-Feed in einem Hordenreaktor durchgeführt werden. Vorzugsweise wird sie mit sauerstoffhaltigem Gas als Co-Feed durchgeführt. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,25 mol/mol, besonders bevorzugt 0,05 bis 0,25 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff mit Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispielen 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert. Zur Regenerierung können auch Rauchgase aus Verbrennungsanlagen mit einem Restsauerstoffgehalt von 2 bis 6 Vol.-%, welche neben Stickstoff und Sauerstoff noch Kohlenstoffoxide, Wasserdampf und geringe Mengen an Kohlenwasserstoffen enthalten, eingesetzt werden.

Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO₂, sowie Leichtsieder (Methan, Ethan, Ethen, Propan und Propen). Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 0 bis 20 Vol.-% 1-Buten, 0 bis 40 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff und 0 bis 15 Vol.-% Kohlenstoffoxide.

Der die erste Dehydrierzone verlassende Produktgasstrom b kann in zwei Teilströme aufgetrennt werden, wobei nur einer der beiden Teilströme den weiteren Verfahrensteilen C bis H unterworfen wird und der zweite Teilstrom in die erste Dehydrierzone zurückgeführt wird. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen C bis G unterworfen werden.

Der nicht-oxidativen katalytischen Dehydrierung wird erfindungsgemäß eine oxidative Dehydrierung (Oxidehydrierung) als Verfahrensteil C nachgeschaltet. Dabei werden im Wesentlichen 1-Buten und 2-Buten zu 1,3-Butadien dehydriert, wobei 1-Buten im Allgemeinen nahezu vollständig abreagiert.

Diese kann grundsätzlich in allen aus dem Stand der Technik bekannten Reaktortypen und Fahrweisen durchgeführt werden, wie beispielsweise im Wirbelbett, im Hordenofen im Festbettrohr- oder -rohrbündelreaktor oder im Plattenwärmetauscherreaktor. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der nicht-oxidativen katalytischen Dehydrierung stammende Produktgasgemisch mit Sauerstoff oder einem sauerstoffhaltigem Gas vermischt. Das sauerstoffhaltige Gas ist wie im Falle der ersten (autothermen) Dehydrierstufe B) bevorzugt Luft oder mit Sauerstoff angereicherte Luft mit einem Sauerstoffgehalt bis 70 Vol.-%, vorzugsweise bis 50 Vol.-%. Das erhaltene sauerstoffhaltige Gasgemisch wird dann der Oxidehydrierung zugeführt.

Die für die Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7.5}K_{0,1}Oₓ + SiO₂) und DE-A 24 40 329 (Mo₁₂BiCO_{4,5}Ni_{2,5}Cr₃P_{0,5}K_{0,1}Oₓ).

Die Stöchiometrie der Aktivmasse einer Vielzahl der für die Oxidehydrierung geeigneten Multimetalloxidkatalysatoren lässt sich unter die allgemeine Formel (I)

Mo₁₂BiₐFe_{b}Co_{c}Ni_{d}CrₑX¹_{f}K_{g}Oₓ (I),

in der die Variablen nachfolgende Bedeutung aufweisen:
- X¹ =: W, Sn, Mn, La, Ce, Ge, Ti, Zr, Hf, Nb, P, Si, Sb, Al, Cd und/oder Mg;
- a =: 0,5 bis 5, vorzugsweise 0,5 bis 2;
- b =: 0 bis 5, vorzugsweise 2 bis 4;
- c =: 0 bis 10, vorzugsweise 3 bis 10;
- d =: 0 bis 10;
- e =: 0 bis 10, vorzugsweise 0,1 bis 4;
- f =: 0 bis 5, vorzugsweise 0,1 bis 2;
- g =: 0 bis 2, vorzugsweise 0,01 bis 1; und
- x =: eine Zahl, die durch die Wertigkeit und Häufigkeit der von Sauerstoff verschiedenen Elemente in (I) bestimmt wird;
subsumieren.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13.75}BiFe₃CO_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sr_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ). Herstellung und Charakterisierung der genannten Katalysatoren sind ausführlich in den zitierten Schriften beschrieben.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als so genannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des Weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung wird im Allgemeinen bei einer Temperatur von 220 bis 490 °C und bevorzugt von 250 bis 450 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Durch die Kopplung der nicht-oxidativen katalytischen, bevorzugt autothermen Dehydrierung mit der oxidativen Dehydrierung der gebildeten n-Butene wird eine sehr viel höhere Ausbeute an Butadien, bezogen auf eingesetztes n-Butan, erhalten. Ferner kann die nicht-oxidative Dehydrierung schonender betrieben werden. Vergleichbare Butadien-Ausbeuten wären mit einer ausschließlich nicht-oxidativen Dehydrierung nur zum Preis deutlich niedrigerer Selektivitäten zu erreichen. Mit einer ausschließlich oxidativen Dehydrierung werden nur geringe n-Butan-Umsätze erzielt.

Der die oxidative Dehydrierung verlassende Produktgasstrom c enthält neben Butadien und nicht umgesetztem n-Butan im Allgemeinen noch Wasserstoff, Kohlenstoffoxide und Wasserdampf. Als Nebenbestandteile kann er noch Sauerstoff, Stickstoff, Methan, Ethan, Ethen, Propan und Propen sowie sauerstoffhaltige Kohlenwasserstoffe, sogenannte Oxygenate, enthalten. Im Allgemeinen enthält er praktisch kein 1-Buten mehr und nur noch geringe Anteile an 2-Buten.

Im Allgemeinen weist der die oxidative Dehydrierung verlassende Produktgasstrom c 2 bis 40 Vol.-% Butadien, 5 bis 80 Vol.-% n-Butan, 0 bis 15 Vol.-% 2-Buten, 0 bis 5 Vol.-% 1-Buten, 5 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 70 Vol.-% Stickstoff, 0 bis 5 Vol.-% Kohlenstoffoxide und 0 bis 10 Vol.-% Oxygenate auf. Oxygenate können beispielsweise Furan, Essigsäure, Maleinsäureanhydrid, Maleinsäure, Propionsäure, Acetaldehyd, Acrolein, Formaldehyd, Ameisensäure und Butyraldehyd sein. Daneben können in Spuren Acetylen, Propin und 1,2-Butadien enthalten sein. Der Produktgasstrom c kann noch geringe Mengen Sauerstoff enthalten. Enthält der Produktgasstrom c mehr als nur geringfügige Spuren Sauerstoff, so wird im Allgemeinen eine Verfahrensstufe zur Entfernung von Rest-Sauerstoff aus dem Produktgasstrom c durchgeführt. Der Rest-Sauerstoff kann sich insoweit als störend auswirken, als er in nach gelagerten Verfahrensschritten als Initiator für Polymerisationsreaktionen wirken kann. Diese Gefahr ist insbesondere bei der destillativen Abtrennung von Butadien (Schritt E)) gegeben und kann dort zu Ablagerungen von Polymeren (Bildung von so genanntem "Popcorn") in der Extraktivdestillationskolonne führen. Vorzugsweise wird die Sauerstoff-Entfernung unmittelbar nach der oxidativen Dehydrierung durchgeführt. Im Allgemeinen wird hierzu eine katalytische Verbrennungsstufe durchgeführt, in der Sauerstoff mit dem in dem Gasstrom c enthaltenen Wasserstoff in Gegenwart eines Katalysators umgesetzt wird. Hierdurch wird eine Verringerung des Sauerstoffgehalts bis auf geringe Spuren erreicht.

Ein geeigneter Katalysator für die Oxidation von Wasserstoff enthält, geträgert auf α-Aluminiumoxid, 0,01 bis 0,1 Gew.-% Platin und 0,01 bis 0,1 Gew.-% Zinn bezogen auf das Gesamtgewicht des Katalysators. Platin und Zinn werden vorteilhaft in einem Gewichtsverhältnis von 1:4 bis 1:0,2 eingesetzt, bevorzugt in einem Verhältnis von 1:2 bis 1:0,5, insbesondere in einem Verhältnis von annährend 1:1. Vorteilhaft enthält der Katalysator 0,05 bis 0,09 Gew.-% Platin und 0,05 bis 0,09 Gew.-% Zinn bezogen auf das Gesamtgewicht des Katalysators. Neben Platin und Zinn können gegebenenfalls Alkali- und/oder Erdalkalimetallverbindungen in Mengen kleiner 2 Gew.-%, insbesondere kleiner 0,5 Gew.-% verwendet werden. Besonders bevorzugt enthält der Aluminiumoxid-Katalysator ausschließlich Platin und Zinn. Der Katalysatorträger aus α-Aluminiumoxid weist vorteilhaft eine BET-Oberfläche von 0,5 bis 15 m²/g auf, bevorzugt 2 bis 14 m²/g, insbesondere 7 bis 11 m²/g. Als Träger wird bevorzugt ein Formkörper eingesetzt. Bevorzugte Geometrien sind beispielsweise Tabletten, Ringtabletten, Kugeln, Zylinder, Sternstränge oder zahnradförmige Stränge mit Durchmessern von 1 bis 10 mm, bevorzugt 2 bis 6 mm. Besonders bevorzugt sind Kugeln oder Zylinder, insbesondere Zylinder.

Alternatiwerfahren zur Entfernung von Rest-Sauerstoff aus dem Produktgasstrom c umfassen das Inkontaktbringen des Produktgasstroms mit einem Gemisch aus Metalloxiden, welches in reduzierter Form Kupfer in der Oxidationsstufe 0 enthält. Daneben enthält ein derartiges Gemisch im Allgemeinen noch Aluminiumoxide und Zinkoxide, wobei der Kupfergehalt üblicher Weise bis zu 10 Gew.-% beträgt. Auf diese Weise ist eine nahezu vollständige Abtrennung von Rest-Sauerstoff möglich. Daneben können weitere Methoden der Entfernung von Sauerstoffspuren zur Anwendung kommen. Beispiele sind die Abtrennung mittels Molsieben oder unter Einsatz von Membranen.

In einer Verfahrensstufe D) wird der Gasstrom c in mindestens einer ersten Kompressionsstufe komprimiert und anschließend abgekühlt, wobei mindestens ein Kondensatstrom d1 enthaltend Wasser auskondensiert und ein Gasstrom d2 enthaltend n-Butan, Butadien, Wasserstoff, Wasserdampf, gegebenenfalls Kohlenstoffoxide und gegebenenfalls Inertgase verbleibt.

Vorzugsweise wird der Gasstrom c vor der ersten Kompressionsstufe auf eine Temperatur im Bereich von 15 bis 60°C abgekühlt. Die Abkühlung erfolgt durch direkten oder indirekten Wärmetausch. Beim direkten Wärmetausch wird rückgeführtes Kondensat mit dem Gasstrom c in Kontakt gebracht. Geeignete Kontaktapparate sind Waschkolonnen, Quenchkolonnen, Venturiwäscher. Gegebenenfalls wird dem Quenchumlaufstrom NaNO₂ zugegeben, um Spuren von Sauerstoff zu entfernen. Gegebenenfalls wird der Quenchumlaufmenge Stabilisator gegen die Bildung von Popcorn, Polymere oder Butadienperoxide zugegeben.

Die Kompression kann ein- oder mehrstufig erfolgen. Insgesamt wird von einem Druck im Bereich von 1,0 bis 4,0 bar auf einen Druck im Bereich von 3,5 bis 8,0 bar komprimiert. Nach jeder Kompressionsstufe folgt eine Abkühlstufe, in der der Gasstrom auf eine Temperatur im Bereich von 15 bis 60 °C abgekühlt wird. Der Kondensatstrom d1 kann somit bei mehrstufiger Kompression auch mehrere Ströme umfassen.

Der Gasstrom d2 besteht im Allgemeinen im Wesentlichen aus C₄-Kohlenwasserstoffen (im Wesentlichen n-Butan und Butadien), Wasserstoff, Kohlendioxid und Wasserdampf. Daneben kann der Strom d2 noch Leichtsieder und Inertgase (Stickstoff) als weitere Nebenkomponenten enthalten. Der Abwasserstrom d1 besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlendioxid.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck : Eintrittsdruck) pro Verdichterstufe liegen je nach Bauart zwischen 1,5 und 3,0.

Gegebenenfalls wird vor dem Verfahrensschritt E) aus dem Gasstrom c Kohlendioxid durch Gaswäsche abgetrennt, wobei ein an Kohlendioxid abgereicherter Gasstrom d2 erhalten wird. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Gasstrom d2 durch ein- oder mehrstufige Verdichtung auf einen Druck im Bereich von 5 bis 25 bar verdichtet.

Es wird ein an Kohlendioxid abgereicherter Gasstrom d2 mit einem CO₂-Gehalt von im Allgemeinen < 100 ppm, vorzugsweise < 10 ppm erhalten.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

In einer Verfahrensstufe E) wird der Gasstrom d2 durch Extraktivdestillation in einen im Wesentlichen aus Butadien bestehenden Produktstrom e1 und einen n-Butan, Wasserstoff, Wasserdampf, gegebenenfalls Kohlenstoffoxide und gegebenenfalls Inertgase enthaltenden Strom e2 aufgetrennt.

Die Extraktivdestillation kann beispielsweise wie in Erdöl und Kohle - Erdgas - Petrochemie Band 34 (8), Seiten 343 - 346 oder Ullmanns Enzyklopädie der Technischen Chemie, Band 9, 4. Auflage 1975, Seiten 1 bis 18 beschrieben durchgeführt werden.

Hierzu wird der Gasstrom d2 mit einem Extraktionsmittel, vorzugsweise ein N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Böden auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mittels flüssigem Kohlenwasserstoffrücklauf, wozu die Kopffraktion zuvor kondensiert wird. Typische Temperaturen am Kopf der Kolonne liegen zwischen 30 und 60 °C. Das Massenverhältnis Extraktionsmittel zu C₄-Produktgasstrom d im Zulauf der Extraktionszone beträgt im allgemeinen 10 : 1 bis 20 : 1.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte cyclische Säureamide verwendet. Besonders vorteilhaft sind Dimethylformamid, Acetonitril, Furfural und insbesondere NMP.

Es können jedoch auch Mischungen dieser Extraktionsmittel untereinander, z. B. von NMP und Acetonitril, Mischungen dieser Extraktionsmittel mit Co-Lösungsmitteln und/oder tert.-Butylether, z. B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

In der Extraktivdestillationskolonne wird ein gasförmiger, zumindest n-Butan, Wasserdampf und Wasserstoff enthaltender Strom e2, der im Allgemeinen über den Kolonnenkopf abgezogen wird, und als Seitenabzugsstrom ein Gemisch aus Extraktionsmittel und Butadien gewonnen. Aus diesem Gemisch kann Butadien nachfolgend als Reinprodukt gewonnen werden. Als Sumpfabzugsstrom wird das Extraktionsmittel, welches noch Butadien und gegebenenfalls Nebenkomponenten (Verunreinigungen) enthält, gewonnen. Der Sumpfabzugsstrom wird, gegebenenfalls nach Durchführung weiterer Aufreinigungsschritten, wieder in die Extraktivdestillation zurückgeführt.

Der Strom e2 kann als weitere Bestandteile noch Butene, Leichtsieder, Kohlenstoffoxide und Inertgase (Stickstoff) enthalten, im Allgemeinen enthält er diese Bestandteile.

Beispielsweise kann die Extraktivdestillation, Isolierung des Rein-Butadiens und Aufreinigung des Extraktionsmittels wie folgt durchgeführt werden: Der Seitenabzugsstrom der Extraktivdestillationskolonne aus Extraktionsmittel und Butadien, der noch Verunreinigungen enthält (Acetylen, Propin, 1,2-Butadien), wird in eine Waschkolonne eingespeist, welche mit frischem Extraktionsmittel beschickt wird. Am Kolonnenkopf der Waschkolonne wird Roh-Butadien, welches beispielsweise 98 Gew.-% Butadien enthält, abgezogen. Der Sumpfabzugsstrom ist mit Acetylen angereichert und wird in die Extraktivdestillation zurückgeführt. Das Roh-Butadien kann als Verunreinigungen Propin und 1,2-Butadien enthalten. Zur Entfernung dieser Verunreinigungen wird das Roh-Butadien einer ersten Reindestillationskolonne zugeführt und über Kopf ein mit Propin angereicherter Butadienstrom abgetrennt. Der Sumpfabzugsstrom, der im Wesentlichen Propin-frei ist, aber noch Spuren von 1,2-Butadien enthält, wird in eine zweite Reindestillationskolonne eingespeist, in der ein im Wesentlichen 1,2-Butadien-freier Rein-Butadienstrom mit einer Reinheit von beispielsweise mindestens 99,6 Gew.-% als Kopfabzugsstrom oder Seitenabzugsstrom im Verstärkungsteil der Kolonne und ein mit 1,2-Butadien angereicherter Sumpfabzugsstrom erhalten werden.

Zur Aufreinigung des Extraktionsmittels wird ein Teil des Extraktionsmittels als Sumpfabzugsstrom aus der Extraktivdestillationskolonne ausgeschleust und wie folgt regeneriert: Die Extraktionslösung wird in eine Desorptionszone mit gegenüber der Extraktionszone vermindertem Druck und/oder erhöhter Temperatur überführt, wobei aus der Extraktionslösung Butadien und vorhandene Acetylen-Spuren desorbiert werden. Die Desorptionszone kann beispielsweise in Form einer Waschkolonne ausgeführt sein, die 5 bis 15, bevorzugt 8 bis 10 theoretische Stufen und eine Rückwaschzone mit beispielsweise 4 theoretischen Stufen aufweist. Diese Rückwaschzone dient zur Rückgewinnung des in der Gasphase enthaltenen Extraktionsmittels mittels flüssigem Kohlenwasserstoffrücklaufes, wozu die Kopffraktion zuvor kondensiert wird oder durch Zugabe von Wasser als Rücklauf. Als Einbauten sind Packungen, Böden oder Füllkörper vorgesehen. Der Druck am Kolonnenkopf beträgt beispielsweise 1,5 bar. Die Temperatur im Kolonnensumpf liegt beispielsweise bei 130 bis 150 °C. Am Kolonnensumpf wird ein im Wesentlichen Acetylen-freies Extraktionsmittel erhalten, welches in die Extraktivdestillationskolonne zurückgeführt wird.

In einer Verfahrensstufe F) wird der Gasstrom e2 in abgekühlt und gegebenenfalls in einer oder mehreren weiteren Kompressionsstufe komprimiert, wobei mindestens ein Kondensatstrom f1 enthaltend n-Butan und Wasser und ein Gasstrom f2 enthaltend n-Butan, Wasserstoff, gegebenenfalls Kohlendioxid und gegebenenfalls Inertgase erhalten werden. Der Kondensatstrom f1 kann in die erste Dehydrierzone zurückgeführt werden.

Die Kompression kann wiederum ein- oder mehrstufig erfolgen. Im Allgemeinen wird insgesamt von einem Druck im Bereich von 3,5 bis 8 bar auf einen Druck im Bereich von 8 bis 40 bar komprimiert. Nach jeder Kompressionsstufe folgt eine Abkühlstufe, in der der Gasstrom auf eine Temperatur im Bereich von 0 bis 60 °C abgekühlt wird. Der Kondensatstrom f1 kann somit bei mehrstufiger Kompression auch mehrere Ströme umfassen. Vorzugsweise wird die Kompression in einer Stufen durchgeführt.

Der Gasstrom f2 enthält im Allgemeinen als wesentliche Komponenten n-Butan, Kohlendioxid und Wasserstoff. Daneben kann er noch Butene, Leichtsieder und Inertgase (Stickstoff) als weitere Nebenkomponenten enthalten. Auch Wasserdampf kann noch in geringen Mengen enthalten sein. Der Kondensatstrom f1 besteht im Allgemeinen zu mindestens 50 Gew.-%, vorzugsweise zu mindestens 70 Gew.-% aus C₄-Kohlenwasserstoffen (im Wesentlichen n-Butan, daneben gegebenenfalls noch Butene) und enthält daneben noch Wasser und im Allgemeinen noch Kohlendioxid, weiterhin kann er noch Leichtsieder und Spuren von Oxygenaten enthalten.

In einer Verfahrensstufe G) wird der Gasstrom f2 in einen Rückführstrom g1 enthaltend n-Butan und einen Abgasstrom g2 enthaltend Wasserstoff und Kohlendioxid durch in Kontakt bringen des Gasstroms f2 mit einem hoch siedenden Absorptionsmittel und anschließender Desorption der in dem Absorptionsmittel gelösten Gasbestandteile aufgetrennt.

In der Verfahrensstufe G) werden die nicht kondensierbaren oder leicht siedenden Gasbestandteile wie Wasserstoff, Sauerstoff, Kohlendioxid, die leicht siedenden Kohlenwasserstoffe (Methan, Ethan, Ethen, Propan, Propen) und gegebenenfalls Stickstoff in einem Absorptions-/Desorptions-Cyclus mittels eines hoch siedenden Absorptionsmittels von den C₄-Kohlenwasserstoffen abgetrennt, wobei ein Rückführstrom g1 erhalten wird, der die C₄-Kohlenwasserstoffe enthält, und ein Abgasstrom g2 erhalten wird. Wird mit einem sauerstoffhaltigen Gas gestrippt, so kann der Rückführstrom g1 größere Mengen Sauerstoff und Inertgase enthalten.

Dazu wird in einer Absorptionsstufe der Gasstrom f2 mit einem inerten Absorptionsmittel in Kontakt gebracht, wobei die C₄-Kohlenwasserstoffe in dem inerten Absorptionsmittel absorbiert werden und ein mit C₄-Kohlenwasserstoffen beladenes Absorptionsmittel und ein die übrigen Gasbestandteile enthaltendes Abgas g2 erhalten werden. Im Wesentlichen sind dies Kohlendioxid und Wasserstoff sowie gegebenenfalls Inertgase. In einer Desorptionsstufe werden die C₄-Kohlenwasserstoffe aus dem Absorptionsmittel wieder freigesetzt.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Die Absorption kann durch einfaches Durchleiten des Stroms f2 durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption der C₄-Kohlenwasserstoffe wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Desorptionsmittels durchgeführt. In einer weiteren Verfahrensvariante wird zusätzlich mit Sauerstoff oder einem sauerstoffhaltigen Gas gestrippt. Dadurch wird zumindest ein Teil des für die autotherme Dehydrierung benötigten Sauerstoffs in den Prozess eingeführt.

Die Abtrennung G) ist im Allgemeinen nicht ganz vollständig, so dass in dem C₄-Kohlenwasserstoffstrom g1 - je nach Art der Abtrennung - noch geringe Mengen oder auch nur Spuren der weiteren Gasbestandteile, insbesondere der leicht siedenden Kohlenwasserstoffe, vorliegen können.

Zur Abtrennung des im Abgasstrom g2 enthaltenen Wasserstoffs kann dieser, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

### Beispiel

Ein n-Butan enthaltender Einsatzgasstrom (4), welcher durch Vereinigung eines Frischgasstroms (1) und der Rückführströme (12) und (15) erhalten wird, wird der ersten, autotherm betriebenen, nicht-oxidativen katalytischen n-Butan-Dehydrierungsstufe (BDH) (17) zugeführt. Zur Bereitstellung der für die endotherme Dehydrierung notwendigen Wärme wird selektiv Wasserstoff verbrannt. Die Verbrennungsluft wird als Strom (2) zugeführt. Um einer Verkokung des Katalysators entgegenzuwirken und die Standzeit des Katalysators zu verlängern wird außerdem Wasserdampf (3) zugegeben. Es wird ein Dehydriergasgemisch (5) erhalten, das nach Austritt aus der autothermen Dehydrierstufe (17) abgekühlt und der zweiten, oxidativen n-Butan-Dehydrierstufe (ODH) (18) zugeführt wird. Der ODH (18) wird ferner ein Sauerstoffstrom (6) zugeführt. Für BDH und ODH wurden basierend auf experimentellen Ergebnissen die in Tabelle 1 wiedergegebenen Umsatzgrade bzw. Selektivitäten angenommen.

**Tabelle 1**

| Reaktionsstufe | Umsatz [%] | Selektivität [%] |
|---|---|---|
| Autotherme Dehydrierung (BDH) | 49,5 (n-Butan) | 97,9 (zu Butenen/Butadien) |
| Oxidative Dehydrierung (ODH) | 100,0 (1-Buten) 92,7(2-Buten) | 95,0 (zu Butadien) |

Aus dem Austrittsgas der Oxidehydrierung (7), welches unter einem Druck von 2,3 bar steht, wird der Restsauerstoff durch katalytische Verbrennung von Wasserstoff entfernt, wobei ein praktisch sauerstofffreier Gasstrom (7a) resultiert. Dazu wird der Gasstrom (7) in einem Reaktor (20) mit einem Katalysator in Kontakt gebracht. Anschließend wird der Gasstrom (7a) abgekühlt und in einem Verdichter (20) auf einen Druck von 4,1 bar verdichtet. Das verdichtete Gas (9) wird abgekühlt und einer Extraktionskolonne (21) zugeführt, wo die Abtrennung des Butadiens (10) unter Verwendung von NMP als Lösungsmittel erfolgt. Der nach der Butadien-Extraktion verbleibende n-Butan reiche Strom (11) wird in einer zweiten Kompressionsstufe (22) auf einen Druck von 10,1 bar verdichtet und auf eine Temperatur von 20 °C abgekühlt, wobei ein Kondensatstrom (12) anfällt, der im Wesentlichen aus n-Butan besteht, und der in die erste Dehydrierstufe zurückgeführt wird. Der verdichtete Gasstrom (13), der im Wesentlichen aus n-Butan, Kohlendioxid und Wasserstoff besteht, wird einer Absorptions/Desorptionsstufe (23) zugeführt, welche unter Verwendung von Tetradecan als Absorptionsmittel betrieben wird. Es wird ein im Wesentlichen aus Kohlendioxid und Wasserstoff bestehendes Abgas (14), welches noch geringe Mengen Leichtsieder enthält, abgetrennt. Die Desorption des in dem Absorptionsmittel Tetradecan gelösten n-Butans erfolgt durch Strippen mit Sauerstoff (16). Es wird auf diese Weise ein im Wesentlichen aus n-Butan und Sauerstoff bestehender Rückführstrom (15), der noch geringe Mengen Kohlendioxid und Leichtsieder enthält, erhalten und in die erste Dehydrierstufe zurückgeführt.

Die Ergebnisse der Simulationsrechnung sind in Tabelle 2 wiedergegeben. Die Zusammensetzung der Stoffströme (1) bis (16) ist in Gewichtsanteilen angegeben.

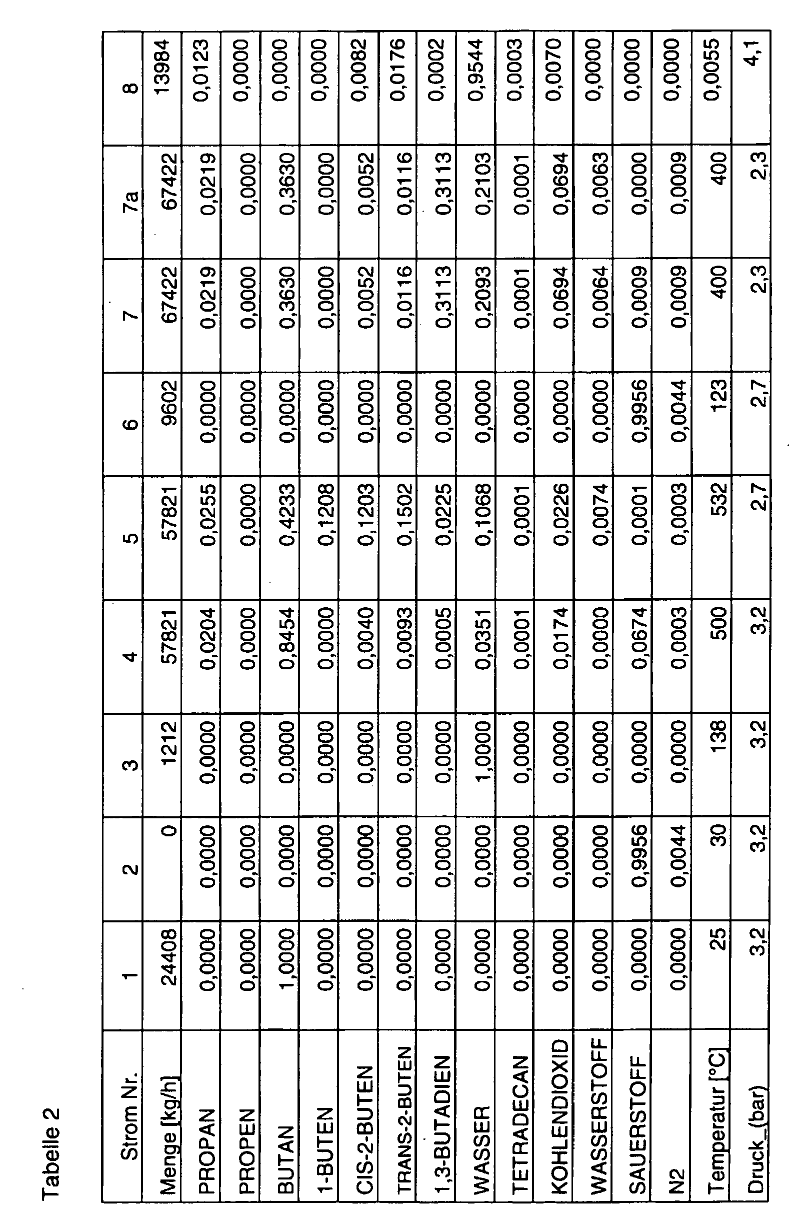

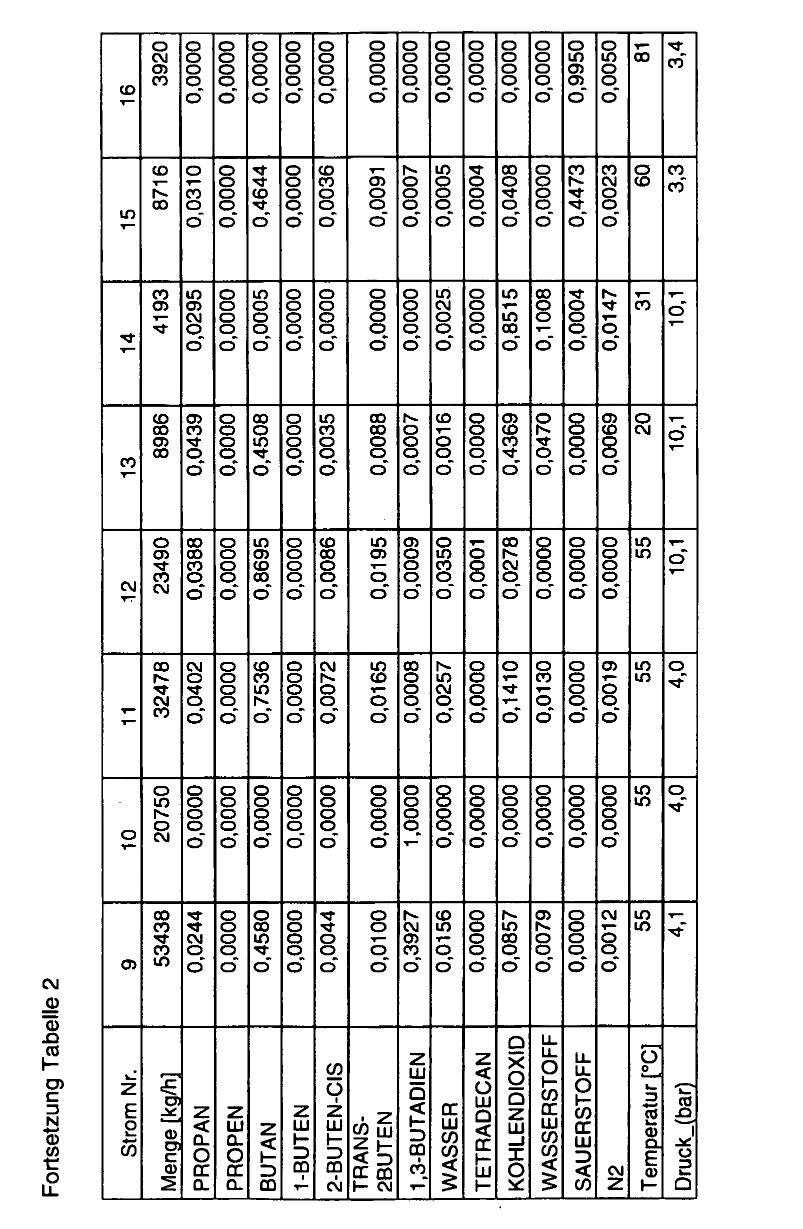

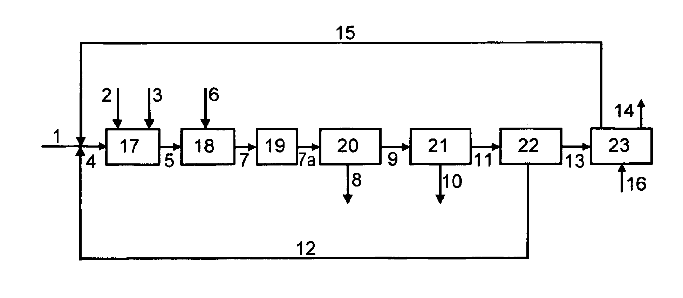

## Patentansprüche

1. Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Buten, Butadien, Wasserstoff, gegebenenfalls Wasserdampf, gegebenenfalls Kohlenstoffoxide und gegebenenfalls Inertgase erhalten wird;
C) Einspeisung des Gasstroms b und eines sauerstoffhaltigen Gases in mindestens eine zweite Dehydrierzone und oxidative Dehydrierung von 1-Buten und 2-Buten, wobei ein Gasstrom c enthaltend n-Butan, Butadien, Wasserstoff, Wasserdampf, gegebenenfalls Kohlenstoffoxide und gegebenenfalls Inertgase erhalten wird;
D) Kompression in mindestens einer ersten Kompressionsstufe und Abkühlung des Gasstroms c, wobei mindestens ein Kondensatstrom d1 enthaltend Wasser und ein Gasstrom d2 enthaltend n-Butan, Butadien, Wasserstoff, Wasserdampf, gegebenenfalls Kohlenstoffoxide und gegebenenfalls Inertgase erhalten wird;
E) Auftrennung des Gasstroms d2 durch Extraktivdestillation in einen im Wesentlichen aus Butadien bestehenden Produktstrom e1 und einen n-Butan, Wasserstoff, Wasserdampf, gegebenenfalls Kohlenstoffoxide und gegebenenfalls Inertgase enthaltenden Strom e2;
F) gegebenenfalls Kompression in einer oder mehreren weiteren Kompressionsstufen und Abkühlung des Gasstroms e2, wobei mindestens ein Kondensatstrom f1 enthaltend n-Butan und Wasser und ein Gasstrom f2 enthaltend n-Butan, Wasserstoff, gegebenenfalls Kohlenstoffoxide und gegebenenfalls Inertgase erhalten werden, und gegebenenfalls Rückführung des Kondensatstroms f1 in die erste Dehydrierzone;
G) Auftrennung des Gasstroms f2 in einen Rückführstrom g1 enthaltend n-Butan und einen Abgasstrom g2 enthaltend Wasserstoff, gegebenenfalls Kohlenstoffoxide und gegebenenfalls Inertgase durch in Kontakt bringen des Gasstroms f2 mit einem hoch siedenden Absorptionsmittel und anschließender Desorption der in dem Absorptionsmittel gelösten Gasbestandteile.

2. Verfahren nach Anspruch 1, **dadurch gekennzeichnet, dass** die nicht-oxidative, katalytische Dehydrierung von n-Butan autotherm unter Einspeisung eines sauerstoffhaltigen Gases durchgeführt wird.

3. Verfahren nach Anspruch 2, **dadurch gekennzeichnet, dass** als sauerstoffhaltiges Gas Luft oder mit Sauerstoff angereicherte Luft eingespeist wird.

4. Verfahren nach Anspruch 2, **dadurch gekennzeichnet, dass** als sauerstoffhaltiges Gas technisch reiner Sauerstoff eingespeist wird.

5. Verfahren nach einer der Ansprüche 1 bis 4, **dadurch gekennzeichnet, dass** der n-Butan enthaltende Einsatzstrom a aus liquefied petroleum gas (LPG) gewonnen wird.

6. Verfahren nach einem der Ansprüche 1 bis 5, **dadurch gekennzeichnet, dass** nach der Stufe C) der im Produktgas der oxidativen Dehydrierung verbliebene Sauerstoff entfernt wird, indem er katalytisch mit Wasserstoff umgesetzt wird.

## Claims

1. A process for preparing butadiene from n-butane, which comprises the steps
A) provision of a feed gas stream a comprising n-butane;
B) feeding of the feed gas stream a comprising n-butane into at least one first dehydrogenation zone and nonoxidative, catalytic dehydrogenation of n-butane to give a gas stream b comprising n-butane, 1-butene, 2-butene, butadiene, hydrogen, possibly water vapor, possibly carbon oxides and possibly inert gases;
C) feeding of the gas stream b and an oxygen-comprising gas into at least one second dehydrogenation zone and oxidative dehydrogenation of 1-butene and 2-butene to give a gas stream c comprising n-butane, butadiene, hydrogen, water vapor, possibly carbon oxides and possibly inert gases;
D) compression in at least one first compression stage and cooling of the gas stream c to give at least one condensate stream d1 comprising water and a gas stream d2 comprising n-butane, butadiene, hydrogen, water vapor, possibly carbon oxides and possibly inert gases;
E) fractionation of the gas stream d2 by extractive distillation to give a product stream e1 comprising essentially butadiene and a stream e2 comprising n-butane, hydrogen, water vapor, possibly carbon oxides and possibly inert gases;
F) If appropriate, compression in one or more further compression stages and cooling of the gas stream a2 to give at least one condensate stream f1 comprising n-butane and water and a gas stream f2 comprising n-butane, hydrogen, possibly carbon oxides and possibly inert gases and, if appropriate, recirculation of the condensate stream f1 to the first dehydrogenation zone;
G) fractionation of the gas stream f2 to give a recycle stream g1 comprising n-butane and an offgas stream g2 comprising hydrogen, possibly carbon oxides and possibly inert gases by contacting of the gas stream f2 with a high-boiling absorption medium and subsequent desorption of the gas constituents dissolved in the absorption medium.

2. The process according to claim 1, wherein the nonoxidative, catalytic dehydrogenation of n-butane is carried out autothermally with introduction of an oxygen-comprising gas.

3. The process according to claim 2, wherein air or air enriched with oxygen is fed in as oxygen-comprising gas.

4. The process according to claim 2, wherein technically pure oxygen is fed in as oxygen-comprising gas.

5. The process according to any of claims 1 to 4, wherein the feed stream comprising n-butane is obtained from liquefied petroleum gas (LPG).

6. The process according to any of claims 1 to 5, wherein the oxygen remaining in the product gas from the oxidative dehydrogenation after step C) is removed by reacting it catalytically with hydrogen.

## Revendications

1. Procédé pour la fabrication de butadiène à partir de n-butane, comprenant les étapes suivantes :
A) on prépare un courant de gaz d'utilisation a contenant du n-butane ;
B) on injecte le courant de gaz d'utilisation a, contenant du n-butane, dans au moins une première zone de déshydrogénation, on effectue une déshydrogénation catalytique non oxydante, ce qui forme un courant de gaz b contenant du n-butane, du 1-butène, du 2-butène, du butadiène, de l'hydrogène, éventuellement, de la vapeur d'eau, éventuellement des oxydes de carbone et, éventuellement, des gaz inertes ;
C) on injecte le courant de gaz b et un gaz contenant de l'oxygène dans au moins une seconde zone de déshydrogénation, et on effectue une déshydrogénation oxydante du 1-butène et du 2-butène pour obtenir un courant de gaz c contenant du n-butane, du butadiène, de l'hydrogène, de la vapeur d'eau, éventuellement des oxydes de carbone et, éventuellement, des gaz inertes ;
D) on comprime le courant de gaz c dans au moins une première étape de compression et on le refroidit de manière à obtenir au moins un courant de condensat d1, contenant de l'eau, et un courant de gaz d2, contenant du n-butane, du butadiène, de l'hydrogène, de la vapeur d'eau, éventuellement des oxydes de carbone et, éventuellement, des gaz inertes ;
E) on sépare le courant de gaz d2 par distillation par extraction en un courant de produit e1 constitué essentiellement de butadiène et un courant e2 comprenant du n-butane, de l'hydrogène, de la vapeur d'eau, éventuellement des oxydes de carbone et éventuellement des gaz inertes ;
F) éventuellement on comprime le courant de gaz e2 dans une ou plusieurs autres étapes de compression, et on le refroidit de manière à obtenir au moins un courant de condensat f1 contenant du n-butane et de l'eau, et un courant de gaz f2 contenant du n-butane, de l'hydrogène, éventuellement, des oxydes de carbone et, éventuellement, des gaz inertes, et, éventuellement on renvoie le courant de condensat f1 dans la première zone de déshydrogénation ;
G) on sépare le courant de gaz f2 en un courant de recyclage g1 comprenant du n-butane et un courant de gaz résiduels g2 comprenant de l'hydrogène, éventuellement des oxydes de carbone et éventuellement des gaz inertes, par mise en contact du courant de gaz f2 avec un agent d'absorption à point d'ébullition élevé et ensuite désorption des constituants gazeux dissous dans l'agent d'absorption.

2. Procédé selon la revendication 1, **caractérisé en ce que** la déshydrogénation non oxydante catalytique du n-butane est effectuée en mode autothermique par injection d'un gaz contenant de l'oxygène.

3. Procédé selon la revendication 2, **caractérisé en ce que** l'on injecte de l'air ou de l'air enrichi en oxygène comme gaz contenant de l'oxygène.

4. Procédé selon la revendication 2, **caractérisé en ce que** l'on injecte de l'oxygène de pureté technique comme gaz contenant de l'oxygène.

5. Procédé selon l'une quelconque des revendications 1 à 4, **caractérisé en ce que** le courant d'utilisation a, contenant du n-butane, provient de gaz de pétrole liquéfié (GPL).

6. Procédé selon l'une quelconque des revendications 1 à 5, **caractérisé en ce que**, après l'étape C), l'oxygène restant dans le gaz produit de la déshydrogénation oxydante est éliminé en le faisant réagir catalytiquement avec de l'hydrogène.
